# EUROPEAN PATENT SPECIFICATION

(11) **EP 2 568 823 B1**
(45) Date of publication and mention of the grant of the patent: **27.04.2016**
(21) Application number: 11720682.1
(22) Date of filing: 09.05.2011
(51) Int. Cl.: A23K 20/00, A23K 20/20, A23K 50/40, C12Q 1/68

(54) **METHODS OF DIAGNOSIS, CONTROL AND PROPHYLAXIS OF INFLAMMATION AND MITIGATION OF INFLAMMATORY CONDITIONS IN FELINES**
VERFAHREN ZUR DIAGNOSE, STEUERUNG UND PROPHYLASE VON ENTZÜNDUNGEN UND ABSCHWÄCHUNG VON ENTZÜNDUNGSERKRANKUNGEN BEI KATZEN
PROCÉDÉS POUR DIAGNOSTIQUER, LIMITER, ET PRÉVENIR L'INFLAMMATION, ET POUR ATTÉNUER DES ÉTATS INFLAMMATOIRES CHEZ LES FÉLINS

(30) Priority: 12.05.2010 US 334078 P
(43) Date of publication of application: 20.03.2013
(73) Proprietor: Hill's Pet Nutrition, Inc., Topeka, KS 66603 (US)
(72) Inventor: AL-MURRANI, Samer, Topeka, Kansas 66614 (US); GAO, Xiangming, Topeka, Kansas 66615 (US); LEE, Junyu, Topeka, Kansas 66614 (US); MALLADI, Sukhaswami, Lawrence, Kansas 66049 (US); FRANTZ, Nolan Zebulon, Meadville, Pennsylvania 16335 (US)
(74) Representative: Daniels, Jeffrey Nicholas
(86) International application number: PCT/US2011/035731
(87) International publication number: WO 2011/143102

(56) References cited:
- WO-A1-2010/009474
- WO-A2-2008/137549
- US-A1- 2009 111 877
- "Research Abstract Program of the 2010 ACVIM Forum", JOURNAL OF VETERINARY INTERNAL MEDICINE, vol. 24, no. 3, 1 May 2010 (2010-05-01), pages 660-795, XP55003635, ISSN: 0891-6640, DOI: 10.1111/j.1939-1676.2010.0521.x

## Description

### Field of the Invention

Described herein are methods of diagnosis, control and prophylaxis of inflammation and mitigation of inflammatory conditions, particularly arthritis and joint pain, in felines, comprising measuring particular inflammatory biomarkers. Further provided is a method to treat or control inflammation comprising administering a diet comprising increased levels of one or more of DHA, EPA, methionine, manganese, vitamin C, vitamin E, and/or L-carnitine. The invention is defined by claims 1-4.

### Background of the Invention

Degenerative joint diseases (DJD) or osteoarthritis (OA) in felines has been less recognized than in canines. However, several authors have estimated the occurrence of appendicular DJD in the feline population to range from 16.5% to 64% of older felines. Little is known about the etiology and predisposing factors of feline DJD. Utilizing genomic and proteomic technologies may help provide insight into the disease process and understand how treatment therapies may aid in the management of this disease.

Osteoarthritis is a chronic, degenerative joint disease that is caused by the progressive inflammation and deterioration of the cartilage, bone, and soft tissue of one or more joints. Rheumatoid arthritis is an autoimmune condition that causes inflammation and damage to the joints. Both are chronic inflammatory conditions. Because the damage to the joints is progressive and largely irreversible, it is desirable to identify and address the inflammatory process proactively. Unfortunately, biomarker expression in blood has proved difficult to correlate with expression in tissue, making diagnosis difficult before the disease leads to severe pain and irreversible tissue damage.

A composition for improving the quality of life of a super senior feline is known from US2009/11187. WO2010/009474 discloses a method of treating an abnormal joint condition in a subject, comprising administering a composition comprising at least one omega-3 fatty acid, at least one glycosaminoglycan, at least one amino sugar, at least one antioxidant, and carnitine or acetylcarnitine to a subject in need thereof.

### Summary of the invention

Six genes that were up-regulated in felines with OA compared to normal geriatric felines were identified. These genes were then measured utilizing the same arthritic felines after consuming the test food. A diet which provides clinical benefit correlating with the reduction in expression of the six genes has also been identified.

Described herein is a method of detecting an inflammatory condition in a feline, comprising measuring blood levels of a gene selected from one or more of INDO, ANXA1, MMP8, CD79a, ODC1, and STXBP2, wherein increased expression is correlated with an inflammatory condition.

Further described is a method of control and/or prophylaxis of inflammation, or mitigation of inflammatory conditions, particularly arthritis and joint pain, in a feline, comprising identifying the condition by measuring blood expression levels of INDO, ANXA1, MMP8, CD79a, ODC1, and/ or STXBP2, and administering a diet comprising increased levels of one or more of DHA, EPA, methionine, manganese, vitamin C, vitamin E, and/or L-carnitine, e.g., for a period of at least two weeks.

The invention provides a composition as defined in claims 1-4 for use in treating or controlling an inflammatory condition in a feline.

### Detailed Description of the Invention

The diet for use in the methods herein includes for example, a feline diet comprising increased levels of one or more of DHA, EPA, methionine, manganese, vitamin C, vitamin E, and/or L-carnitine. e.g., comprising DHA+EPA 0.25 - 5% on a dry weight basis, for example a diet comprising, on a dry weight basis:
DHA + EPA: 0.5-2.5%
Methionine: 1-3% mg/kg,
Manganese: 50-200 ppm
Vitamin C: 75-1000mg/kg
Vitamin E: 500 - 2500 mg/kg
L-carnitine: 100-1500 mg/kg
for example a diet having approximately the nutritional composition of the test diet of Example 1, e.g., having ingredients in the approximately amounts identified in Table 1, +/-10% on a dry weight basis.

### Example 1 - Effect of diet on inflammatory biomarkers in arthritic felines

This study was conducted to evaluate the effect of a test food on whole blood gene expression profiles when fed to felines with appendicular degenerative joint disease (DJD). Thirty-one domestic short hair felines (initial weight, 4.2 ± 1.27 kg, age, 12.1 ± 2.98 years) with lameness and radiographic changes consistent with DJD were included in the study. All felines were fed a control food for 28 days followed by a test food. The test food contained increased levels of DHA and EPA, methionine, manganese, Vitamins C and E, and L-carnitine. Whole blood samples were collected on the last day of the control food and after 14 days on the test food. After consuming the test food for 14 days, felines with DJD had decreased expression of the 6 genes (INDO, ANXA1, MMP8, CD79a, ODC1, and STXBP2), which were identified to be up-regulated in DJD versus healthy geriatric felines. These genes are associated with cartilage degradation, B cell function, polyamine synthesis, and protein trafficking. In summary, feeding the test food to felines with DJD resulted in reversal of the gene expression patterns previously observed in the blood of felines with DJD versus healthy geriatric felines after 14 days.

Thirty-one neutered/ spayed domestic short hair felines were identified for this study. Felines with lameness were radiographed to confirm changes consistent with degenerative joint disease. Felines were considered otherwise healthy by physical exam and serum chemistry profiles. All felines were immunized against feline panleukopenia, calici and herpes viruses, and rabies, and none had chronic systemic disease on the basis of results of physical examination, complete blood count determination, serum biochemical analyses, urinalysis, and fecal examination for parasites. Felines were housed with 10 - 12 other felines and food was continuously available throughout the day until their daily caloric requirements were consumed. Felines were housed in spacious rooms with natural light that varied with seasonal changes. Felines experienced behavioral enrichment through interactions with each other, by daily interaction and play time with caretakers, large windows and sun porches to watch the natural landscape and access to toys. All felines were fed a maintenance control food for 28 days before day 0 collection, followed by the test food. Blood was drawn and collected into PAXgene tubes and stored at -20°C until evaluation.

The Partek® GS (Partek Inc., St. Charles, MO) for Gene Expression Data software (Partek Incorporated, 12747 Olive Blvd., Suite 205, St. Louis, Missouri 63141, U.S.A. http://www.partek.com/partekgs_geneexpression) was used for data analysis. The Robust Multichip Average (RMA) algorithm (1) was used for background adjustment, normalization, and probe-level summarization of the GeneChip® samples. The ANOVA analysis was performed to find significant differentially expressed genes between any two groups with a minimal FDR control at 0.1 and a fold change of 1.3 in each direction. Furthermore, the false discovery rate threshold of 0.1 (means that 10% of observations are due to chance) was chosen as the minimum level of acceptable statistical significance. Similar cutoff values were used the RT-PCR analysis of the 74 selected genes for verification of the microarray data.

**Table 1: Nutritional values of control and test diet**

| **Nutrients, 100% Dry Matter Basis** | **Control maintenance food** | **Test food** |
|---|---|---|
| Crude Protein, % | 34.4 | 32.3 |
| Fat, % | 19.4 | 20.7 |
| Crude Fiber, % | 2.1 | 2.6 |
| Insoluble fiber, % | 5.8 | 7.6 |
| Soluble fiber, % | 0.1 | 1.7 |
| Calcium, % | 0.95 | 1.04 |
| Phosphorus, % | 0.86 | 0.78 |
| Potassium, % | 0.6 | 1.0 |
| Sodium, % | 0.4 | 0.3 |
| Magnesium, % | 0.07 | 0.09 |
| Chloride, % | 0.75 | 0.96 |
| Linoleic Acid, % | 3.1 | 3.6 |
| Alpha linolenic acid, % | 0.13 | 0.26 |
| DHA, % | <0.01 | 0.28 |
| EPA, % | <0.01 | 0.38 |
| Lysine, % | 1.5 | 2.7 |
| Methionine, % | 0.92 | 1.54 |
| Manganese, ppm | 15 | 97 |
| Vitamin E, IU/kg | 104 | 1215 |
| Vitamin C, ppm | 0 | 174 |
| Taurine, ppm | 1230 | 2857 |
| Carnitine, ppm | 21 | 504 |

**Table 2: RT-PCR analysis of genomic differences in felines with osteoarthritis compared to healthy geriatric felines**

| | **Day 14 / Day 0** | | |
|---|---|---|---|
| **Gene name** | **Gene Symbol** | **Fold Change** | **P-value** |
| CD79A binding protein 1 | CD79A | -2.4 | 0.04 |
| Annexin A1 | ANXA1 | -17.0 | 0.01 |
| Orinthine Decarboyxlase 1 | ODC1 | -2.3 | 0.01 |
| Indoleamine pyrrole 2,3 dioxygenase | INDO | -3.2 | 0.09 |
| Syntax binding protein 2 | STXBP2 | -2.7 | 0.06 |
| Matrix metalloproteinase 8 | MMP8 | -19.2 | 0.03 |

Based on the day 0 comparison of felines with OA and healthy geriatric felines, 6 genes that were significantly up-regulated in OA felines were identified. After switching the OA felines to a test food, these 6 genes (CP79a, INDO, ANXA1, ODC1, STXBP2, and MMP8) were down-regulated after 14 days. In effect, these genes now appeared more like the expression of that found in the normal geriatric felines. The results of the current study showed the 6 genes identified as significantly up-regulated in felines with OA versus normal geriatric felines were down-regulated after consuming a test food. These specific genes may play an essential role in the development of OA or DJD or the underlying arthritic condition. This data may provide genomic support for the efficacy of the test food in arthritic felines. In agreement with clinical and activity responses measured, arthritic felines fed the test food had gene expression more similar to normal geriatric felines after consuming the test food.

## Claims

1. A composition for use in treating or controlling an inflammatory condition in a feline, which composition consists of, on a dry weight basis:
crude protein: 29.07 - 35.53 %;
fat: 18.63 - 22.77 %;
crude fiber: 2.34 - 2.86 %;
insoluble fiber: 6.84 - 8.36 %;
soluble fiber: 0.963-1.177%;
calcium: 0.936 -1.144 %;
phosphorus: 0.702 - 0.858 %;
potassium: 0.9 -1.1 %;
sodium: 0.27 - 0.33 %;
magnesium: 0.081- 0.099 %;
chloride: 0.864 -1.056 %;
linoleic acid: 3.24 - 3.96 %;
alpha linolenic acid: 0.234 - 0.286 %;
DHA: 0.252 - 0.308 %;
EPA: 0.342 - 0.418 %;
lysine: 2.43 - 2.97 %;
methionine: 1.386 -1.694 %;
manganese: 87.3 -106.7 ppm;
vitamin E:1093.5 -1336.5 IU/kg;
vitamin C:156.6 -191.4 ppm;
taurine: 2571.3 - 3142.7 ppm; and
carnitine: 453.6 - 554.4 ppm.

2. The composition of claim 1 for the use of claim 1 wherein the composition consists of, on a dry matter basis:
Crude Protein: 32.3 %
Fat: 20.7 %
Crude Fiber: 2.6 %
Insoluble fiber: 7.6 %
Soluble fiber: 1.7 %
Calcium: 1.04%
Phosphorus: 0.78%
Potassium: 1.0 %
Sodium: 0.3 %
Magnesium: 0.09 %
Chloride: 0.96 %
Linoleic Acid: 3.6 %
Alpha linolenic acid: 0.26 %
DHA: 0.28 %
EPA: 0.38 %
Lysine: 2.7 %
Methionine: 1.54%
Manganese: 97 ppm
Vitamin E: 1215 IU/kg
Vitamin C: 174 ppm
Taurine: 2857 ppm
Carnitine: 504 ppm.

3. The composition for use of any of the foregoing claims wherein the condition to be controlled or treated is osteoarthritis.

4. The composition for use of any preceding claim, wherein the use comprises a step of detecting the inflammatory condition in the feline prior to treating or controlling the inflammatory condition by measuring blood levels of a gene selected from one or more of INDO, ANXA1, MMP8, ODC1, and STXBP2, wherein increased expression is correlated with an inflammatory condition.

## Patentansprüche

1. Zusammensetzung zur Verwendung bei der Behandlung oder Kontrolle eines Entzündungszustands bei einer Katze, wobei die Zusammensetzung auf einer Trockensubstanzbasis besteht aus:
Rohprotein: 29,07 % - 35,53 %;
Fett: 18,63 % - 22,77 %;
Rohfaser: 2,34 % - 2,86 %;
unlöslicher Faser: 6,84 % - 8,36 %;
löslicher Faser: 0,963 % - 1,177 %;
Calcium: 0,936 % - 1,144 %;
Phosphor: 0,702 % - 0,858 %;
Kalium: 0,9 % - 1,1 %;
Natrium: 0,27 % - 0,33 %;
Magnesium: 0,081 % - 0,099 %;
Chlorid: 0,864 % - 1,056 %;
Linolsäure: 3,24 % - 3,96 %;
α-Linolensäure: 0,234 % - 0,286 %;
DHA: 0,252 % - 0,308 %;
EPA: 0,342 % - 0,418 %;
Lysin: 2,43 % - 2,97 %;
Methionin: 1,386 % - 1,694 %;
Mangan: 87,3 ppm - 106,7 ppm;
Vitamin E: 1093,5 IE/kg - 1336,5 IE/kg;
Vitamin C: 156,6 ppm - 191,4 ppm;
Taurin: 2571,3 ppm - 3142,7 ppm; und
Carnitin: 453,6 ppm - 554,4 ppm.

2. Zusammensetzung nach Anspruch 1 zur Verwendung nach Anspruch 1, wobei die Zusammensetzung auf einer Trockensubstanzbasis besteht aus:
Rohprotein: 32,3 %
Fett: 20,7 %
Rohfaser: 2,6 %
unlöslicher Faser: 7,6 %
löslicher Faser: 1,7 %
Calcium: 1,04 %
Phosphor: 0,78 %
Kalium: 1,0 %
Natrium: 0,3 %
Magnesium: 0,09 %
Chlorid: 0,96 %
Linolsäure: 3,6 %
α-Linolensäure: 0,26 %
DHA: 0,28 %
EPA: 0,38 %
Lysin: 2,7 %
Methionin: 1,54 %
Mangan: 97 ppm
Vitamin E: 1215 IE/kg
Vitamin C: 174 ppm
Taurin: 2857 ppm
Carnitin: 504 ppm.

3. Zusammensetzung zur Verwendung nach einem der vorangehenden Ansprüche, wobei der zu kontrollierende oder behandelnde Zustand Osteoarthritis ist.

4. Zusammensetzung zur Verwendung nach einem der vorangehenden Ansprüche, wobei die Verwendung einen Schritt zum Nachweis des Entzündungszustands bei der Katze vor der Behandlung oder Kontrolle des Entzündungszustands durch Messen der Blutspiegel eines Gens umfasst, das aus einem oder mehr von INDO, ANXA1, MMP8, ODC1 und STXBP2 ausgewählt ist, wobei eine erhöhte Expression mit einem Entzündungszustand korreliert ist.

## Revendications

1. Composition à utiliser pour traiter ou contrôler une affection inflammatoire chez un félin, ladite composition comprenant, en poids à l'état sec :
protéines brutes : 29 ,07 - 35,53 % ;
matières grasses : 18,63 - 22,77 % ;
fibres brutes : 2,34 - 2,86 % ;
fibres insolubles : 6,84 - 8,36 % ;
fibres solubles : 0,963 - 1,177 % ;
calcium : 0,936 - 1,144 % ;
phosphore : 0,702 - 0,858 % ;
potassium : 0,9 - 1,1 % ;
sodium : 0,27 - 0,33 % ;
magnésium : 0,081 - 0,099 % ;
chlorure : 0,864 - 1,056 % ;
acide linoléique : 3,24 - 3,96 % ;
acide alpha-linolénique : 0,234 - 0,286 % ;
DHA : 0,252 - 0,308 % ;
EPA : 0,342 - 0,418 % ;
lysine : 2,43 - 2,97 % ;
méthionine : 1,386 - 1,694 % ;
manganèse : 87,3 - 106,7 ppm ;
vitamine E : 1093,5 - 1336,5 UI/kg ;
vitamine C : 156,6 - 191,4 ppm ;
taurine : 2571,3 - 3142,7 ppm ; et
carnitine : 453,6 - 554,4 ppm.

2. Composition selon la revendication 1, à utiliser selon la revendication 1, dans laquelle la composition comprend, en poids à l'état sec :
protéines brutes : 32,3 %
matières grasses : 20,7 %
fibres brutes : 2,6 %
fibres insolubles : 7,6 %
fibres solubles : 1,7 %
calcium : 1,04 %
phosphore : 0,78 %
potassium : 1,0 %
sodium : 0,3 %
magnésium : 0,09 %
chlorure : 0,96 %
acide linoléique : 3,6 %
acide alpha-linolénique : 0,26 %
DHA : 0,28 %
EPA : 0,38 %
lysine : 2,7 %
méthionine : 1,54 %
manganèse : 97 ppm
vitamine E : 1215 Ul/kg
vitamine C : 174 ppm
taurine : 2857 ppm
carnitine : 504 ppm.

3. Composition à utiliser selon l'une quelconque des revendications précédentes, dans laquelle l'affection devant être contrôlée ou traitée est l'arthrose.

4. Composition à utiliser selon l'une quelconque des revendications précédentes, dans laquelle l'utilisation comprend l'étape qui consiste à détecter l'affection inflammatoire chez le félin avant de traiter ou de contrôler l'affection inflammatoire en mesurant les taux sanguins d'un gène sélectionné parmi le groupe constitué par un ou plusieurs des gènes INDO, ANXA1, MMP8, ODC1, et STXBP2, dans laquelle une expression accrue est associée à une affection inflammatoire.
